Europäisches Patentamt

European Patent Office

Office européen des brevets

(19)

(11) Veröffentlichungsnummer: 0 049 905

A1

(12) **EUROPÄISCHE PATENTANMELDUNG**

(21) Anmeldenummer: 81108310.4

(22) Anmeldetag: 14.10.81

(51) Int. Cl.³: **G 01 J 3/50**
**A 61 B 1/24**

(30) Priorität: 14.10.80 DE 3038786

(43) Veröffentlichungstag der Anmeldung:
21.04.82 Patentblatt 82/16

(84) Benannte Vertragsstaaten:
AT BE CH DE FR GB IT LI LU NL SE

(71) Anmelder: Fraunhofer-Gesellschaft zur Förderung der
angewandten Forschung e.V.
Leonrodstrasse 54
D-8000 München 19(DE)

(72) Erfinder: Schief, Alfred, Dr.
Reinhl-Schneider-Strasse 48
D-7500 Karlsruhe(DE)

(72) Erfinder: Bretschie, Jürgen, Dr.
Rudolf-Diesel-Strasse 4
D-6904 Eppelheim b. Heidelberg(DE)

(72) Erfinder: Mutschler, Hartmut, Dipl.-Ing.
Rintheimer Strasse 19
D-7500 Karlsruhe(DE)

(72) Erfinder: Reuter, Peter
Hirschstrasse 27
D-7500 Karlsruhe(DE)

(72) Erfinder: Schulte, Willi, Prof.Dr.med. Zentrum für
Zahn- Mund- und Kieferheilkunde Universtät
D-7400 Tübingen(DE)

(72) Erfinder: Lukas, Dieter, Dipl.-Phys. Zentrum für
Zahn- Mund- und Kieferheilkunde Universtät
D-7400 Tübingen(DE)

(54) Einrichtung zur Messung der Farbe des Zahnfleisches.

(57) Die Erfindung betrifft eine Einrichtung zur Messung der Farbe des Zahnfleisches und ist besonders für die Beurteilung von Zahnfleischerkrankungen geeignet. Sie sieht vor, daß das Licht einer Lichtquelle (5) über ein Lichtkabel (2) einer Sonde (1) zugeführt wird und am Austrittsort das Zahnfleisch beleuchtet. Das reflektierte Licht wird über ein zweites rückführendes Lichtkabel (3) in mehrere Wellenlängenbereiche mittels Filter (16 - 18) gewertet und photoelektrischen Bauelementen (19 - 21) zugeführt. Die Signale werden dann ausgewertet in einer elektronischen Einrichtung (22).

Fig. 1

FRAUNHOFER-GESELLSCHAFT ZUR FÖRDERUNG
DER ANGEWANDTEN FORSCHUNG E.V.
Leonrodstr. 54
8000  München 19

**0049905**

80/13691-IITB

## Einrichtung zur Messung der Farbe des Zahnfleisches

Die Schwere und der Verlauf von Zahnbetterkrankungen (Parodontopathien) lassen sich sehr häufig vom Entzündungsgrad des Zahnfleisches ableiten. Da der Entzündungsgrad mit der Durchblutung des betroffenen Gewebebezirkes korreliert, wird erfindungsgemäß vorgeschlagen, diese Durchblutung optisch aus der Farbe des Zahnfleisches zu ermitteln.

Beim heutigen Stand der Technik versucht man mit wenig reproduzierbaren, schlecht dokumentierbaren und subjektiven Einflüssen unterworfenen Verfahren den Entzündungsgrad des Zahnfleisches zu ermitteln. Eine Möglichkeit ist die visuelle Beurteilung der Zahnfleischfarbe durch den behandelnden Zahnarzt. In einem weiteren Verfahren wird durch einen mechanischen Reiz in der Zahnfleischfurche oder in der Zahnfleischtasche künstlich eine Blutung erzeugt; das Ausmaß der blutbedeckten Flächen und damit indirekt die Geschwindigkeit des Blutaustrittes werden vom Arzt visuell bewertet und als Maß für die Schwere der Entzündung herangezogen. Auch die Sulcus-Fluid-Fließrate dient zur Bestimmung des Entzündungsgrades; hierbei werden Teststreifen in die Zahnfleischtasche (Sulcus) eingebracht und nach drei Minuten Liegedauer die Menge des Zahnfleischexsudates gemessen.

Der Einsatz heutiger technischer Geräte zur Farbmessung für die vorliegende Aufgabe scheitert an ihrer Größe und schlechten Handhabbarkeit im Bereich der Mundhöhle und an den unvermeidbaren Bewegungen des Patienten, die eine definierte Festlegung eines genügend kleinen Meßfleckens auf dem Zahnfleisch verhindern.

Mit dem Verfahren gemäß der Erfindung ist eine schnelle, leicht handhabbare, objektive, reproduzierbare und dokumentierbare Messung der Zahnfleischfarbe und damit ein Rückschluß auf die Zahnfleischdurchblutung möglich.

Die Erfindung wird im folgenden anhand des schematischen Bildes 1 beispielhaft beschrieben.

Ein wesentlicher Bestandteil des Meßverfahrens ist eine leichte und schlank geformte Sonde 1, mit der eine definierte Beleuchtung des Zahnfleisches an der gewünschten Stelle und eine Sammlung des vom Zahnfleisch zurückkommenden Lichtes erfolgt. Durch Verwendung von flexiblen, dünnen Lichtleiterbündeln 2 und 3 gelingt es, die Sonde in einer ergonomisch günstigen Form zu gestalten und die voluminösen und schweren Teile des Gerätes in einem getrennten Gehäuse 4 unterzubringen, das mit der Sonde durch die Lichtleiterbündel verbunden ist.

Eine Lichtquelle 5, beispielsweise eine Glühlampe, erzeugt weißes Licht, das durch eine Linse 6 auf die geräteseitige Endfläche 7 des Lichtleiterbündels geworfen wird. Es tritt an der sondenseitigen Endfläche 8 des Lichtleiterbündels 2 wieder aus und beleuchtet das in Bild 1 nicht gezeichnete Zahnfleisch. Das vom Zahnfleisch zurückgeworfene Licht tritt über die sondenseitige Endfläche 9 in das Lichtleiterbündel 3 ein und wird zum Gerät 4 geführt. Zur Farbmessung wird das Lichtleiterbündel 3 in drei Teilbündel 10,11 und 12 aufgeteilt, so daß an jeder der geräteseitigen Endflächen 13, 14 und 15 der Teilbündel ein definierter Teil des vom Lichtleiterbündel 3 aufgenommenen Lichtes austritt. Die Farbfilter 16, 17 und 18 dienen zur Bewertung des aus den Teilbündeln austretenden Lichtes entsprechend den Normspektralwertfunktionen der Lichtmeßtechnik. Die gefilterten Teillichtströme werden mit den photoelektrischen Bauelementen 19, 20 und 21 in proportionale elektrische Signale umgewandelt. Die elektronische Einrichtung 22, die beispielsweise aus Analog-Digital-Wandlern und einem Mikrorechner besteht, ermittelt aus den elektrischen Signalen der photoelektrischen Bauelemente die Koordinaten der Farbe des Zahnfleisches im Farbdreieck nach den aus der Farbmetrik bekannten Beziehungen. Eine Umrechnung in Farbton und Farbsättigung ist ebenfalls möglich. Diese Koordinaten werden durch eine Anzeige 23 an den Zahnarzt ausgegeben und mit einem Drucker 24 registriert. Die nachfolgend beschriebenen Maßnahmen dienen einzeln oder gemeinsam zur Verbesserung der Wirkungsweise des beschriebenen Verfahrens.

Um die Abhängigkeit der Messung von der Richtung der Sonde gegenüber der Zahnfleischoberfläche zu reduzieren, wird das sondenseitige Ende der Sonde 2 in mehrere Teilbündel aufgeteilt. Diese Teilbündel werden symmetrisch zum Bündel 3 angeordnet. In Bild 2 ist ein Blick auf die Endfläche 9 des Bündels 3 und der darum herum angeordneten Endflächen 25 bis 30 der sechs Teilbündel des Bündels 2 dargestellt. In entsprechender Weise kann auch das Bündel 3 aufgeteilt und um das Bündel 2 angeordnet werden. In diesem Fall können auch anstelle des Bündels 3 sechs durchgehende Teilbündel verwendet werden; am geräteseitigen Ende werden dann jeweils zwei gegenüberliegende Teilbündel zusammen einem der Filter 16 bis 18 und dem dazugehörenden photoelektrischen Bauelement zugeführt. Weiterhin können zwei Lichtleiterbündel verwendet werden, deren voneinander optisch isolierte Einzelfasern in einem gemeinsamen Ende zusammengefaßt sind, wobei die Einzelfasern jedes Bündels gleichmäßig über die Austrittsfläche verteilt sind.

Durch linsenförmiges Schleifen der sondenseitigen Austrittsflächen 8 oder 9 wird eine optische Abbildung des Lichtleiterquerschnittes auf die Zahnfleischoberfläche bewirkt, durch die die Größe des Meßflecks verringert werden kann.

Anstelle der drei parallel arbeitenden optischen Kanäle, die aus je einem der Teilbündel 10, 11 oder 12 der Filter 16, 17 oder 18 und der photoelektrischen Bauelemente 19, 20 oder 21 bestehen, kann auch ein einziger optischer Kanal verwendet werden, in dem die einzelnen Normspektralwertfunktionen zeitlich hintereinander gebildet werden. Bild 3 zeigt den prinzipiellen Aufbau. Hinter dem geräteseitigen Ende des Lichtleiters 3 werden die den drei Normspektralwertfunktionen entsprechenden Filter durch eine mechanische Einrichtung zeitlich nacheinander in den Strahlengang geführt; beispielsweise sind diese Filter in Löchern einer kreisförmigen Scheibe 31 angebracht, die von einem Synchronmotor 32 gleichförmig gedreht wird. Auf diese Weise wird das photoelektrische Bauelement 33 zeitlich nacheinander mit den Lichtstromanteilen beaufschlagt, die den drei Normspektralwertfunktionen entsprechen. Die angeschlossene eletronische Einrichtung speichert die dazugehörigen Werte und führt, wie bei Bild 1 beschrieben, ihre weitere Verrechnung aus.

Da die Farbwerte des Zahnfleisches nur einen Teil des Farbdreieckes abdecken, können anstelle genauer Realisierungen der Normspektralwertfunktionen Filter verwendet werden, die diese Funktionen nur annähern. Eine Eichung der Meßeinrichtung wird dann zur Erhöhung der Meßgenauigkeit mit einem Farbmuster durchgeführt, das einer mittleren Zahnfleischfarbe entspricht.

Um zeitlich langsam verlaufende, beispielsweise durch Temperaturänderungen verursachte Schwankungen der Eigenschaften der Lichtquelle 5 und der Empfindlichkeiten der photoelektrischen Bauelemente 19, 20, 21 oder 33 zu eliminieren, wird während der Meßpausen eine automatische Eichung des Gerätes durchgeführt. Dazu wird die Sonde in eine Halterung eingeführt, in der das der Zahnfleischfarbe entsprechende Eichfarbmuster an der dem Sondenende gegenüber liegenden Stelle angebracht ist. Der in der elektronischen Einrichtung 22 enthaltene Prozessor gleicht in dieser Stellung die Empfindlichkeit der den Normspektralwertfunktionen entsprechenden Meßkanäle ab.

- 5 -

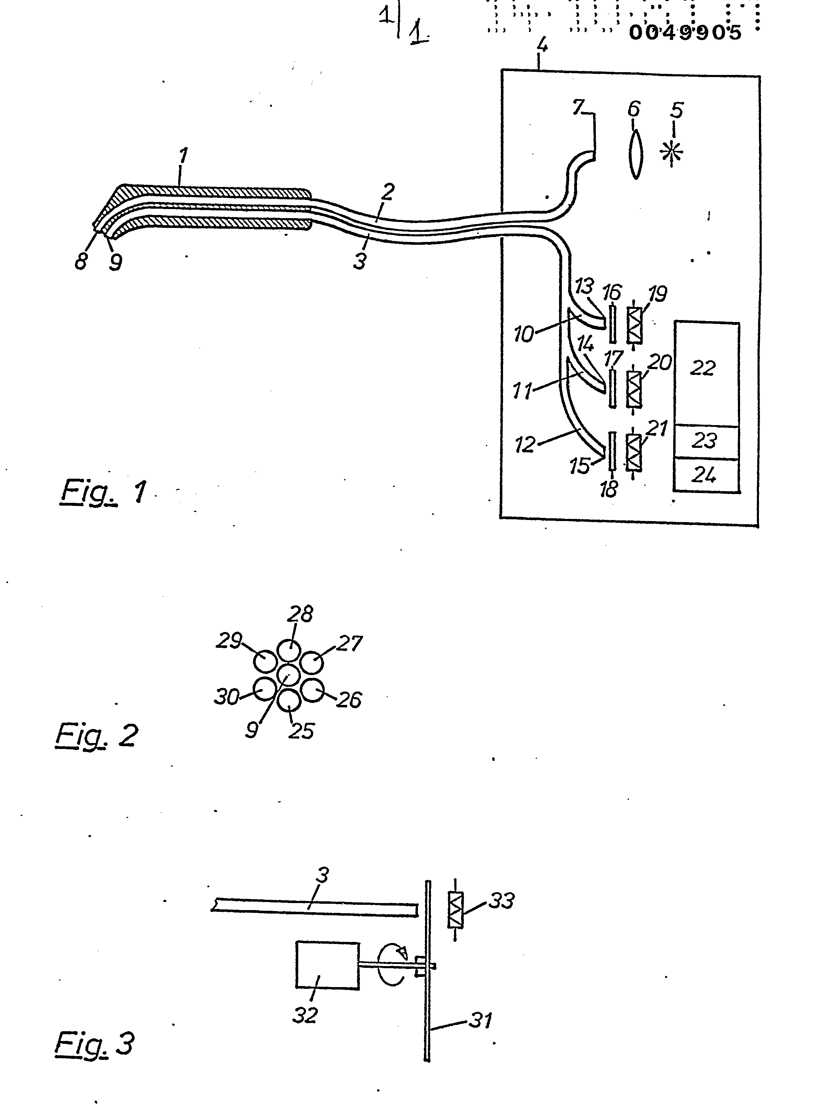

Patentansprüche

1. Einrichtung zur Messung der Farbe des Zahnfleisches zur objektiven Beurteilung des Entzündungs- grades,

   dadurch gekennzeinet,

   daß

   a)  das Licht einer Lichtquelle (5) über ein erstes flexibles Lichtleiterbündel (2) einer leicht handhabbaren Sonde (1) zugeführt wird,

   b)  das von der Oberfläche des Zahnfleisches zurückgeworfene Licht über ein zweites, ebenfalls in der Sonde endendes, flexibles Lichtleiterbündel (3) aufgenommen ,

   c)  dieses Licht über mehrere Filter (16-18) von einem oder mehreren photoelektrischen Bauelementen (19, 20, 21, 33) aufgenommen und in ein elektrisches Signal umgewandelt und

   d)  in einer elektronischen Einrichtung (22) verarbeitet wird.

2. Einrichtung nach Anspruch 1, dadurch gekennzeichnet, daß das zweite Lichtleiterbündel (3) am geräteseitigen Ende in drei Teilbündel (10 - 12) aufgespalten wird und der Lichtstrom jedes Teilbündels über einen optischen Bewertungsfilter (16 - 18) entsprechend den drei Normspektralwertfunktionen einem photoelektrischen Bauelement (19 - 21) zugeführt wird.

3. Einrichtung nach Anspruch 1,

d a d u r c h   g e k e n n z e i c h n et,

daß das sondenartige Ende eines der beiden Lichtleiterbündel in mehrere Teilbündel (25 - 30) aufgespalten wird, deren Endflächen symmetrisch um
die Endfläche des nicht aufgespaltenen Lichtleiterbündels (9) angeordnet werden.

4. Einrichtung nach Anspruch 1 und 3,

d a d u r c h   g e k e n n z e i c h n e t ,

daß zwischen der geräteseitigen Endfläche des zweiten Lichtleiterbündels (3) und einem photoelektrischen Bauelement (33) eine bewegliche mechanische
Einrichtung (31, 32), z.B. eine rotierende Scheibe
(31) angeordnet ist, die nacheinander optische Bewertungsfilter in den Strahlengang führt und weiterhin eine elektronische Einrichtung (22) vorgesehen ist, die die zu den einzelnen Bewertungsfiltern gehörenden Teile des von dem photoelektrischen
Bauelement abgegebenen Signales speichert und anschließend verarbeitet.

5. Einrichtung nach den Ansprüchen 1 bis 4,

d a d u r c h   g e k e n n z e i c h n e t ,

daß die sondenseitigen Austrittsflächen (8, 9, 13,
14, 15) der Lichtleiterbündel zur verkleinernden
Abbildung des Faserquerschnittes linsenförmig geschliffen sind.

Fig. 1

Fig. 2

Fig. 3

**Europäisches Patentamt**

## EUROPÄISCHER RECHERCHENBERICHT

Nummer der Anmeldung

EP 81 10 8310

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe. soweit erforderlich. der maßgeblichen Teile | betrifft Anspruch | |
| A | <u>DE - A - 1 498 513</u> (AMERICAN OPTICAL CY.) <br> * Seiten 8,9,20; Figuren 1,3,4 * | 1,3 | G 01 J 3/50 <br> A 61 B 1/24 |
| | -- | | |
| A | <u>DE - A - 2 264 433</u> (SIEMENS) <br> * Seite 4; Figur 1 * | 1,2 | |
| | -- | | |
| A | <u>DE - A - 2 741 913</u> (OXIMETRIX) <br> * Seiten 10,12-14; Figuren 1,3, 8 * | 1,3 | RECHERCHIERTE SACHGEBIETE (Int. Cl.³) |
| | -- | | |
| A | MEDICAL AND BIOLOGICAL ENGINEERING Band 6, Nr. 4, 1968, Seiten 409-413, Pergamon Press Oxford, G.B. P. EDHOLM et al.: "Tissue identification during needle puncture by reflection spectrophotometry" <br> * Seite 410 * | 1,4 | G 01 J 3/50 <br> A 61 B 1/24 <br> A 61 C 1/08 <br> G 01 N 21/55 |
| | -- | | |
| A | <u>US - A - 3 136 310</u> (R. MELTZER) <br> * Spalte 2, Zeilen 3-16; Spalte 3, Zeilen 26-37; Figuren 2-5 * | 1,4,5 | KATEGORIE DER GENANNTEN DOKUMENTE |
| | ---- | | X: von besonderer Bedeutung allein betrachtet <br> Y: von besonderer Bedeutung in Verbindung mit einer anderen Veröffentlichung derselben Kategorie <br> A: technologischer Hintergrund <br> O: nichtschriftliche Offenbarung <br> P: Zwischenliteratur <br> T: der Erfindung zugrunde liegende Theorien oder Grundsätze <br> E: älteres Patentdokument. das jedoch erst am oder nach dem Anmeldedatum veröffentlicht worden ist <br> D: in der Anmeldung angeführtes Dokument <br> L. aus andern Gründen angeführtes Dokument |
| | | | &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 20-01-1982 | BOEHM |

EPA form 1503.1  06.78